# EUROPEAN PATENT APPLICATION

(11) **EP 3 378 445 A1**
(43) Date of publication of application: **26.09.2018**
(21) Application number: 17162648.4
(22) Date of filing: 23.03.2017
(51) Int. Cl.: A61F 2/46

(54) **BONE GRINDER**

(71) Applicant: Ronvig Dental Mfg. A/S, 8721 Daugaard (DK)
(72) Inventor: Rønvig, Jørn, 8721 Daugaard (DK)
(74) Representative: Høiberg P/S

(57) **Abstract**

The present invention relates to a bone grinder, comprising a housing (1) comprising a bone inlet and a grinding chamber, a drum-shaped cutting tool (2) rotatably mounted in the grinding chamber and a drive shaft (5) engaged with the cutting tool, the bone grinder configured to have a gearing of at least 1:1.5 between the cutting tool and the drive shaft. The gearing in the bone grinder enables manual operation without exhaustive effort which brings other advantages such as easier and more thorough cleaning.

## Description

The present invention relates to a device for grinding bone into small pieces.

### Background of invention

Bone grafting, or transplanting of bone tissue, is a reliable and validated surgical procedure where bone material is used to assist the growth of new bone. Bone grafting is possible because bone tissue, unlike most other tissues, has the ability to regenerate completely if provided the space into which to grow. This requires a very small fracture space or some sort of scaffold to do so. As native bone grows, it will generally replace the graft material completely, resulting in a fully integrated region of new bone.

During dental surgery, bone grafting is often used to assist the growth of new bone. Dental implants require bones underneath them for support and proper integration into the mouth. Therefore, bone grafting is used when building up bone around implants placed in tooth sockets after tooth extraction and when filling bone defects after removing the root of a tooth or removal of impacted teeth. For dental surgery it is often necessary to process the bone in the form of crushing or grinding prior to bone grafting surgery. This is achieved by processing the bone graft in a bone grinder, where a larger piece of bone is cut into smaller pieces.

There are generally three different types of bone grafting depending on the source of the bone used for the procedure. The source may be the patient's own body (autologous), cadaveric bone (allograft) or a synthetic material similar to bone. Autologous bone is typically harvested from intra-oral sources such as the chin or extra-oral sources such as the iliac crest, the fibula, the ribs and the mandible. The chin offers a large amount of cortico-cancellous autograft and easy access among all the intraoral sites. It can be easily harvested in the office settings under local anaesthesia. Proximity of the donor and recipient sites reduce operative time and cost. Added advantages include convenient surgical access, low morbidity, elimination of hospital stay, minimal donor site discomfort and avoidance of cutaneous scars.

In addition to dental surgery, bone grafting surgery is also used to assist bone growth in other contexts, e.g. to fuse joints to prevent movement, repair broken bones that have bone loss, and repair broken bone that has not yet healed.

### Summary of invention

As mentioned earlier, a bone grinder is thus often used to prepare bone grafts prior to or during a surgical procedure. A bone grinder is preferably manually operated, as this is more practical in the sterile environment of an operating room. A manually operated bone grinder may be completely sterilised prior to surgery as there is no motor, wires or batteries in the grinder. The bone grinder should be easy to operate, meaning that assembly and disassembly should be straightforward and the grinding process should be performed without the need of significant effort. It should also be possible to vary the size of the bone pieces produced by the grinder. Furthermore, the bone grinder should be easy to clean after use. Finally, in order to optimally utilise the bone material, as little bone as possible should be wasted, especially for autologous bone grafts. It is therefore desirable to have a bone grinder that is easily manually operated, efficiently collects the ground bone, can produce bone pieces of various sizes and offers easy cleaning and sterilization.

This is achieved by the presently disclosed bone grinder comprising a housing comprising a bone inlet and a grinding chamber and a cutting tool, preferably drum-shaped. The cutting tool is preferably rotatably mounted in the grinding chamber. A drive shaft may be engaged with the cutting tool. In a preferred embodiment the bone grinder is configured to have a gearing of at least 1:1.5 between the cutting tool and the drive shaft.

The presently disclosed bone grinder provides a means for grinding bone material in a manual fashion without the need for exhaustive effort by the person operating the bone grinder. The effort needed for operating the bone grinder may be adjusted by changing the gear ratio between the drive shaft and the cutting tool according to the needs and preferences of the persons operating the bone grinder. Manual operation means that there are no motors or wires in the bone grinder. The entire bone grinder may therefore be sterilized in an appropriate manner, thus improving the sanitation of the grinding process.

### Description of drawings

In the following the invention is further described with respect to a number of drawings. The drawings are exemplary and are not to be construed as limiting to the invention.
- Fig. 1: shows a side view of one embodiment of the presently disclosed bone grinder.
- Fig. 2: is a side view illustration of the cutting tool of fig. 1 with the bone receptacle inserted in one end and the drive shaft engaging the annular gear at the other end.
- Fig. 3: shows another side view of the cutting tool of fig. 1 with the drive shaft engaged with the gearing at one end and the bone receptacle outside the cutting tool.
- Fig. 4: shows a top view of the bone receptacle, cutting tool and drive shaft of fig. 1 in a disassembled configuration.
- Fig. 5: is a different view of the bone receptacle, cutting tool and drive shaft of fig. 1 where the annular gear on the cutting tool is also visible.
- Fig. 6: shows an illustration of an example of the gearing between the drive shaft and the annular gear on the cutting tool of fig. 1.

### Detailed description of the invention

In a preferred embodiment of the invention the cutting tool is replaceable allowing the bone to be ground into pieces of varying size and shape and/or allowing bone of different type/hardness to be ground. Furthermore, the cutting tool is preferably surface hardened for increased durability. This may be obtained from a low-temperature surface-hardening process, in which a hardened zone containing nitrogen and carbon is established. Nitrogen adds increased surface hardness while carbon bridges the gap to the softer core and a smooth hardness profile is created. In an exemplary embodiment the cutting tool is surface hardened by the company Expanite.

In a further embodiment, the bone grinder further comprises a cover for the bone inlet, configured for extending into the inlet for pushing unground bone towards the cutting tool. The cover for the bone inlet is preferably shaped ergonomically such that manually pushing bone into the grinder is easy and without pain or discomfort.

In another embodiment the axis defined by the bone inlet is offset from the rotational axis of the cutting tool. In yet another embodiment, the offset angle, defined as the angle between the axis defined by the tangent of the bone inlet at the point where the bone inlet meets the cutting tool, and the line crossing the rotational axis of the cutting tool and the point where the bone inlet meets the cutting tool, is greater than zero. The offset angle may be at least 2°, or at least 4°, or at least 6°, or at least 8°, or at least 10°, or at least 12°, or at least 14°, or at least 16°, or at least 18°, or at least 20°.

In a preferred embodiment the gearing comprises an annular gear located at the cutting tool. In another embodiment the gearing comprises a spur gear located at the cutting tool. Furthermore, the gearing preferably comprises a spur or pinion gear located at the drive shaft. In a preferred embodiment the gearing is configured such that the bone grinder can be manually operated. The bone grinder may be configured to have a gearing of at least 1:2, or at least 1:3, or at least 1:4, or at least 1:5, or at least 1:6, or at least 1:7, or at least 1:8 between the cutting tool and the drive shaft. In a clarifying example, the gear on the drive shaft has 6 teeth and the gear on the cutting tool has 18 teeth, which means that in this example, the gearing is 1:3 between the cutting tool and the drive shaft and the drive shaft therefore has to make three revolutions for the cutting tool to make one revolution.

In a further embodiment the bone grinder further comprises an end cap for mounting on the housing, thereby covering the opening for the grinding chamber. This end cap is preferably suitable for collecting ground bone.

In a further embodiment the bone grinder further comprises a bone receptacle mounted in the cutting tool for collecting ground bone. Preferably at least a part of the bone receptacle is cylindrical such that it fits concentrically inside the cutting tool. Additionally, the bone receptacle should comprise an opening towards the cutting tool allowing ground bone material to enter the receptacle. The bone receptacle improves the collection of the ground bone material by preventing it from dropping to the other side of the cutting tool at the bottom of the grinding chamber. In a preferred embodiment the bone receptacle is furthermore fixed relative to the housing in order to ensure that the opening faces the bone inlet at all times for optimal collection of the bone material. Said fixation of the bone receptacle may be provided by a notch and groove in the housing and the bone receptacle, respectively, or a notch and groove in the bone receptacle and the housing, respectively. The bone receptacle is preferably closed off at the end towards the drive shaft and open at the end towards the end cap. This allows for the bone material to be directed into the end cap and thereby collected herein.

In one embodiment the bone grinder is fabricated from stainless steel and preferably the bone grinder is fabricated from surgical stainless steel such as type 316L. Such materials are less prone to corrosion, meaning that use and cleaning of the bone grinder is less likely to cause corrosion damage on the bone grinder. Additionally, the bone grinder can withstand harsher cleaning agents if needed.

In a preferred embodiment the bone grinder further comprises a handle for manual operation of the bone grinder. In one embodiment the handle is mounted on the drive shaft at the opposite end of the gear end. Preferably the power source for the bone grinder is manual power. Manual power is preferred for the bone grinder as this means that there are no electric components in the bone grinder. This is more practical and hygienic for an operating room as this enables sterilization of the entire bone grinder.

It is conceivable that the bone grinder may be powered by an electric motor in a way such that the bone grinder may be cleaned and sterilized. Therefore, in another embodiment the bone grinder further comprises an electric motor as the power source. The power for the electric motor may be provided by a battery.

In another embodiment the bone grinder is oriented such that the rotational axis of the cutting tool is horizontal during operation. In yet another embodiment the bone grinder is oriented such that the rotational axis of the cutting tool is not horizontal during operation, which allows the ground bone material to enter the end cap during grinding.

### Detailed description of the drawings

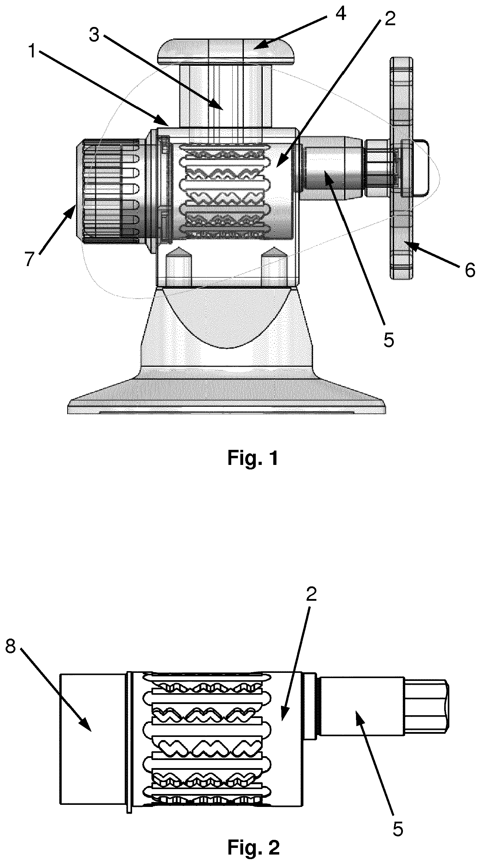

Fig. 1 shows a side view of one embodiment of the presently disclosed bone grinder in an assembled state. The housing 1 is shown in a transparent fashion to reveal the parts inside the grinder. Inside the grinding chamber is the cutting tool 2. Through the top extends the bone inlet 3 with the cover 4 for the inlet inserted into the inlet. At one end of the grinding chamber is the end cap 7 and at the other end the drive shaft 5 is engaged with the cutting tool 2. A handle 6 for manual operation of the grinder is attached to the drive shaft 5.

Fig. 2 is a side view of the cutting tool 2, bone receptacle 8 and drive shaft 5 in an assembled configuration. The gearing at the drive shaft 5 is engaged with the annular gearing in the cutting tool 2 and the bone receptacle 8 is inserted into the cutting tool 2 at the opposite end.

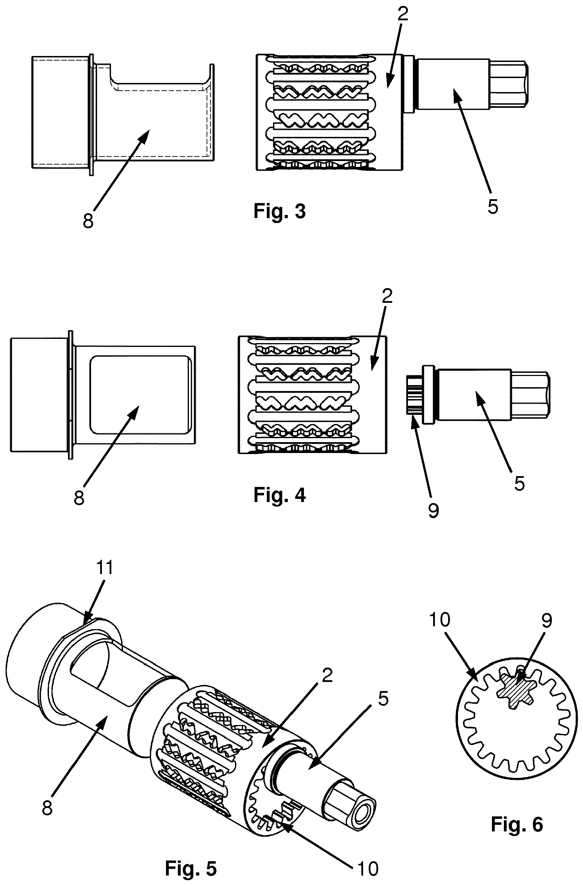

Fig. 3 is another side view of the cutting tool 2 with the drive shaft 5 engaged with the gearing at one end and the bone receptacle 8 outside the cutting tool 2. In this drawing the opening in the bone receptacle 8 is seen in the top part. The bone receptacle 8 in this example has a narrow end that fits concentrically inside the cutting tool 2 and a collar with a diameter larger than the inside diameter of the cutting tool 2.

Fig. 4 shows a top view of the bone receptacle 8, cutting tool 2 and drive shaft 5 in a disassembled configuration. This drawing also shows the spur or pinion gear 9 at one end of the drive shaft 5.

Fig. 5 is another view of the bone receptacle 8, cutting tool 2 and drive shaft 5 where the annular gear 10 on the cutting tool 2 is also visible. The groove 11 in the bone receptacle 8 prevents the bone receptacle 8 from rotating relative to the grinder when in operation such that the opening in the bone receptacle 8 is always facing the bone inlet. In this example the groove is merely a planar part on the collar.

Fig. 6 shows an example of the gearing between the pinion gear 9 on the drive shaft 5 and the annular gear 10 on the cutting tool 2. In this example, the gear on the cutting tool 2 has eighteen teeth and the gear on the drive shaft 5 has six teeth, which means that the gearing is 1:3 between the cutting tool 2 and the drive shaft 5.

### Further details of the present disclosure

The present disclosure may be described by the following items:
1. A bone grinder, comprising:
   - a housing comprising a bone inlet and a grinding chamber;
   - a drum-shaped cutting tool rotatably mounted in the grinding chamber; and
   - a drive shaft engaged with the cutting tool,
   the bone grinder configured to have a gearing of at least 1:1.5 between the cutting tool and the drive shaft.
2. The bone grinder according to item 1, wherein the cutting tool is replaceable allowing the bone to be ground into pieces of varying size and shape and/or allowing bone of different type/hardness to be ground.
3. The bone grinder according to any of the preceding items, wherein the cutting tool is surface hardened for increased durability.
4. The bone grinder according to any of the preceding items, further comprising a cover for the bone inlet, configured for extending into the inlet for pushing unground bone towards the cutting tool.
5. The bone grinder according to item 4, wherein the cover for the bone inlet is shaped ergonomically such that manually pushing bone into the grinder is easy and without pain or discomfort.
6. The bone grinder according to any of the preceding items, wherein the axis defined by the bone inlet is offset from the rotational axis of the cutting tool.
7. The bone grinder according to any of the preceding items, wherein the offset angle, defined as the angle between:
   i. the axis defined by the tangent of the bone inlet at the point where the bone inlet meets the cutting tool, and
   ii. the line crossing the rotational axis of the cutting tool and the point where the bone inlet meets the cutting tool,
   is greater than zero.
8. The bone grinder according to item 7, wherein the offset angle is at least 2°, or at least 4°, or at least 6°, or at least 8°, or at least 10°, or at least 12°, or at least 14°, or at least 16°, or at least 18°, or at least 20°.
9. The bone grinder according to any of the preceding items, wherein the gearing comprises an annular gear located at the cutting tool.
10. The bone grinder according to any of the preceding items, wherein the gearing comprises a spur gear located at the cutting tool.
11. The bone grinder according to any of the preceding items, wherein the gearing comprises a spur or pinion gear located at the drive shaft.
12. The bone grinder according to any of the preceding items, further comprising an end cap for mounting on the housing, thereby covering the opening for the grinding chamber.
13. The bone grinder according to item 12, wherein the end cap is suitable for collecting ground bone.
14. The bone grinder according to any of the preceding items, further comprising a bone receptacle mounted in the cutting tool for collecting ground bone.
15. The bone grinder according to item 14, wherein at least a part of the bone receptacle is cylindrical such that it fits concentrically inside the cutting tool.
16. The bone grinder according to any of items 14 to 15, wherein the bone receptacle comprises an opening towards the cutting tool allowing ground bone material to enter the receptacle.
17. The bone grinder according to any of items 14 to 16, wherein the bone receptacle is fixed relative to the housing.
18. The bone grinder according to item 17, wherein said fixation is provided by a notch and groove in the housing and the bone receptacle, respectively, or a notch and groove in the bone receptacle and the housing, respectively.
19. The bone grinder according to any of the preceding items, wherein the bone grinder is fabricated from stainless steel.
20. The bone grinder according to any of items 1 to 18, wherein the bone grinder is fabricated from surgical stainless steel such as type 316L.
21. The bone grinder according to any of the preceding items, further comprising a handle for manual operation of the bone grinder.
22. The bone grinder according to item 21, wherein the handle is mounted on the drive shaft opposite the gear end.
23. The bone grinder according to any of the preceding items, wherein the power source is manual power.
24. The bone grinder according to any of the preceding items, further comprising an electric motor as the power source.
25. The bone grinder according to item 24, wherein the power for the electric motor is provided by a battery.
26. The bone grinder according to any of the preceding items, wherein the bone grinder is configured to have a gearing of at least 1:2, or at least 1:3, or at least 1:4, or at least 1:5, or at least 1:6, or at least 1:7, or at least 1:8 between the cutting tool and the drive shaft.
27. The bone grinder according to any of the preceding items, wherein the gearing is configured such that the bone grinder can be manually operated.
28. The bone grinder according to any of the preceding items, wherein the bone grinder is oriented such that the rotational axis of the cutting tool is horizontal during operation.
29. The bone grinder according to any of items 1 to 27, wherein the bone grinder is oriented such that the rotational axis of the cutting tool not horizontal during operation, which allows the ground bone material to enter the end cap during operation.

## Claims

1. A bone grinder, comprising:
- a housing comprising a bone inlet and a grinding chamber;
- a drum-shaped cutting tool rotatably mounted in the grinding chamber; and
- a drive shaft engaged with the cutting tool,
the bone grinder configured to have a gearing of at least 1:1.5 between the cutting tool and the drive shaft.

2. The bone grinder according to claim 1, wherein the cutting tool is replaceable allowing the bone to be ground into pieces of varying size and shape and/or allowing bone of different type/hardness to be ground.

3. The bone grinder according to any of the preceding claims, further comprising a cover for the bone inlet, configured for extending into the inlet for pushing unground bone towards the cutting tool.

4. The bone grinder according to any of the preceding claims, wherein the axis defined by the bone inlet is offset from the rotational axis of the cutting tool.

5. The bone grinder according to any of the preceding claims, wherein the gearing comprises an annular gear located at the cutting tool.

6. The bone grinder according to any of the preceding claims, wherein the gearing comprises a spur or pinion gear located at the drive shaft.

7. The bone grinder according to any of the preceding claims, further comprising an end cap for mounting on the housing, thereby covering the opening for the grinding chamber.

8. The bone grinder according to claim 7, wherein the end cap is suitable for collecting ground bone.

9. The bone grinder according to any of the preceding claims, further comprising a bone receptacle mounted in the cutting tool for collecting ground bone.

10. The bone grinder according to claim 9, wherein at least a part of the bone receptacle is cylindrical such that it fits concentrically inside the cutting tool.

11. The bone grinder according to any of claims 9 to 10, wherein the bone receptacle comprises an opening towards the cutting tool allowing ground bone material to enter the receptacle.

12. The bone grinder according to any of claims 9 to 11, wherein the bone receptacle is fixed relative to the housing.

13. The bone grinder according to any of the preceding claims, further comprising a handle for manual operation of the bone grinder.

14. The bone grinder according to any of the preceding claims, wherein the power source is manual power.

15. The bone grinder according to any of the preceding claims, wherein the bone grinder is configured to have a gearing of at least 1:3 between the cutting tool and the drive shaft.
